# EUROPEAN PATENT SPECIFICATION

(11) **EP 3 200 632 B1**
(45) Date of publication and mention of the grant of the patent: **07.06.2023**
(21) Application number: 14789516.3
(22) Date of filing: 03.10.2014
(51) Int. Cl.: A24F 40/40, A24F 40/30, A24F 40/10, A24B 15/167

(54) **ELECTRONIC NICOTINE DELIVERY SYSTEM**
ELEKTRONISCHES NIKOTINABGABESYSTEM
SYSTÈME ÉLECTRONIQUE D'ADMINISTRATION DE NICOTINE

(43) Date of publication of application: 09.08.2017
(73) Proprietor: Fertin Pharma A/S, 7100 Vejle (DK)
(72) Inventor: NIELSEN, Bruno Provstgaard, DK-7120 Vejle Øst (DK)
(74) Representative: Patentgruppen A/S
(86) International application number: PCT/DK2014/050313
(87) International publication number: WO 2016/050245

(56) References cited:
- WO-A1-2013/116558
- DE-U1-202013 105 420
- US-A1- 2014 060 556
- US-A1- 2014 166 028
- US-A1- 2014 261 488

## Description

### Field of invention

The invention relates to electronic nicotine delivery systems.

### Background of the invention

Electronic nicotine delivery systems have become increasingly popular. This comparably new way of delivering nicotine to a user is interesting because it may diminish some of the adverse effects of smoking cigarettes. In this context there is at constant need for improving such devices.

An electronic nicotine delivery system comprising the features of the preamble of claim 1 is known from DE202013105420U.

### Summary of the invention

The invention relates to an electronic nicotine delivery system comprising the features of claim 1.

It has surprisingly been established that pH-controlling agent may be present in aerosols.

One significant advantage of the invention may be that nicotine uptake from the oral cavity of the user may be increased. The present inventor has surprisingly discovered that a significant part of the aerosolized nicotine often settles in the oral cavity of the user before reaching the lungs. Nicotine uptake in the oral cavity may take place through the oral mucosa, but is often not very effective. Therefore, significant parts of the nicotine settling in the oral cavity end up in the gastrointestinal system when swallowed instead of being absorbed into the blood through the oral mucosa. However, by promoting a pH-value in the oral cavity which facilitates more effective nicotine uptake via the oral mucosa, the degree of uptake of nicotine from the oral cavity may be increased. Thereby, it may be possible to reduce the concentration of nicotine in the container arrangement CA, as the nicotine may be absorbed more efficiently.

The pH-controlling agent thus may increase the absorption of nicotine through the oral mucosa. Typically, a fraction of the nicotine from the nicotine container may end up in the oral cavity instead of the lungs. This is a neglected problem related to electronic nicotine delivery systems. Nicotine delivered to the oral cavity may typically be transported via saliva to the gastro-intestinal system, thereby contributing only very little to a desired rapid increase of nicotine concentration in the blood stream. However, if the nicotine is delivered via the oral cavity through the oral mucosa, some effect of the nicotine not reaching the lungs is obtained. Typical natural pH-levels of the oral cavity may not provide an effective uptake of nicotine through the oral mucosa. However, the pH-controlling agent, such as a buffering agent, for adjusting the pH-level in the oral cavity may significantly improve nicotine absorption. Therefore, when the pH-controlling agent for adjusting the pH-level in the oral cavity is delivered together with the nicotine-containing aerosols, a much more effective delivery system may be obtained.

The present inventor surprisingly discovered that the effectiveness of delivering nicotine to a user with an electronic nicotine delivery system may, according to the invention, be improved by adding pH controlling agents to the nicotine solution comprised in the container arrangement.

It has been found that embodiments of the invention increase the uptake of nicotine by a user by at least 2% or even 5%, such as 10% by weight of the total amount of nicotine delivered via the electronic nicotine delivery system when compared to electronic nicotine delivery system not comprising pH-controlling agent in the container arrangement.

Accordingly, it has also been established that embodiments of the invention may be used to deliver a target amount of nicotine to a user by using a lower nicotine concentration in the container arrangement combined with pH-controlling agent, when compared to the nicotine concentration necessary to deliver the same target amount, but without pH-controlling agent.

The improved utilization of nicotine from the electronic nicotine delivery system in these embodiments may be desirable for several reasons. For example, the cost of nicotine implies savings when using smaller amounts, the toxicity of the content in the container arrangement may be lowered due to less nicotine content, the pH-controlling agent may stabilize the nicotine solution to obtain more homogeneous solutions and thereby more homogeneous aerosols with respect to nicotine concentration in the aerosol.

In the context of the present invention the term *"mass median aerodynamic diameter (MMAD)"* is to be understood as the aerodynamic diameter at which 50% of the particles by mass are larger and 50% of the particles by mass are smaller, i.e. the media diameter when evaluating by mass. The aerodynamic diameter of an irregular particle may be defined as the diameter of a spherical particle with a density of 1000 kg/m³ (kilos per cubic meter) and the same settling velocity as the irregular particle.

In the context of the present invention the term "*aerosol*" should be understood as suspension of fine particles in gas, typically a suspension of liquid particles in gas, such as air. Individual aerosols constituents may e.g. be referred to as droplets or particles. Typically, aerosols particles may be associated with certain sizes; however, in the context of the present invention, the term aerosol may refer to particles having a diameter of up to 100 micrometer. General examples of aerosols may be fog or smoke.

In the context of the present invention the term "*atomizer*" should be understood as a device comprising a number of parts, the atomizer being arranged for reducing a liquid to a fine spray of droplets, i.e. a device which transforms a liquid into aerosols. One example of an atomizer may be a device that forces a liquid out of a very small hole so that it becomes a fine spray. A further example is a device that uses heating, such as resistive heating, to evaporate a liquid that may form aerosol upon condensation.

In the context of the present invention the term "*power supply*" should be understood as any electrical portable power source, such as batteries, fuel cells etc.

According to an advantageous embodiment of the invention, the power supply comprises a rechargeable battery.

According to an advantageous embodiment of the invention, the pH-controlling agent is selected to optimize the nicotine uptake via the oral mucosa of a user when the pH-controlling agent comprised in an aerosol produced by the atomizer arrangement is delivered to the oral cavity of a user.

One advantage of the above embodiment may be that a more effective uptake of the nicotine delivered to the oral cavity, which accounts for a large part of the nicotine that does not reach the lungs. Since nicotine may be a relatively expensive substance, a more effective utilization of the nicotine by means of the electronic nicotine delivery system may be a significant advantage. Furthermore, avoiding a very high nicotine concentration to compensate for a lower nicotine uptake, since handling of high nicotine concentration mixtures or liquid may be disadvantageous.

According to an advantageous embodiment of the invention, the pH-controlling agent is selected to establish a pH of above 7 in the saliva in the oral cavity of a user.

Nicotine uptake via oral mucosa is promoted by basic pH. Some users of electronic nicotine delivery systems may naturally have an acidic environment in the oral cavity and even a small shift of pH towards basic values may be effective to take up nicotine via the oral mucosa that would otherwise be swallowed.

According to an advantageous embodiment of the invention, the pH-controlling agent is selected to establish a pH of from about 7.5 to about 10 in the saliva in the oral cavity of a user.

By establishing a pH-value in the above mentioned range in the oral cavity, the uptake of nicotine through the oral mucosa may be significantly increased.

According to an embodiment of the invention the pH-controlling agent is selected to establish a pH of from about 7.5 to about 9.5 in the saliva in the oral cavity of a user or from about 7.5 to about 9.0, such as from about 8.0 - 9.0.

By establishing a pH-value in the above mentioned range in the oral cavity, the uptake of nicotine through the oral mucosa may be increased.

According to an advantageous embodiment of the invention, oral cavity the pH-controlling agent comprises an acidic pH-controlling agent, such as an acidic buffering agent.

The use of an acidic pH-controlling agent may advantageously promote that nicotine containing aerosols are transported to the lungs of a user.

According to an advantageous embodiment of the invention, the container arrangement comprises a nicotine container comprising nicotine and an additive container comprising pH-controlling agent.

According to an advantageous embodiment of the invention, the additive container comprises flavoring.

According to an advantageous embodiment of the invention, said pH-controlling agent comprises a buffering agent.

According to the invention, the content of said nicotine container and/or said additive container comprises buffering agent in the amount of ½ to 5% by weight of the total content of said nicotine container and/or said additive container, such as 1 to 4 %, such as 2 to 5 %, such as 3 to 5 %, such as 3 to 4 %, such as 1 to 3 %.

In an embodiment of the invention, the buffering agent is selected from the group consisting of a carbonate, including bicarbonate or sesquicarbonate, glycerinate, phosphate, glycerophosphate, acetate, glyconate or citrate of an alkali metal, such as potassium or sodium, e.g. trisodium and tripotassium citrate, or ammonium, tris buffer, amino acids, and mixtures thereof.

In an embodiment of the invention, the buffering agent comprises sodium carbonate, sodium bicarbonate or any combination thereof.

In an embodiment of the invention, the buffering agent comprises sodium carbonate, sodium bicarbonate or potassium carbonate.

According to a preferred embodiment of the invention, the buffering agent comprises sodium carbonate.

In connection to the above, it should be understood that said buffering agent may advantageously be in the form of a liquid solution or suspension so as to facilitate the administration of said buffering agent.

In an embodiment of the invention, upon mastication of said chewing gum tablet in an oral cavity, dissolution of said pH control agent and said pharmaceutically active ingredient begin, such that said oral cavity has a salivary pH that is above pKa of said pharmaceutically active ingredient.

According to an advantageous embodiment of the invention, the pH-controlling agent is a buffering agent.

According to an advantageous embodiment of the invention, the atomizer comprises a heating element.

Contrary to every expectation, it has been established that a pH-controlling agent, such as a buffering agent, may even be incorporated into aerosols produced by means of a heating element without losing all the effect of the pH-controlling agent during the heating-invoked evaporation. Thus, it turns out that it is possible to evaporate the pH-controlling agent together with nicotine by means of a heater, thereby making it possible to apply in a heating-based delivery system. An important benefit of this is that it is now possible to apply more attractive and compact technologies for the delivery of nicotine.

According to an advantageous embodiment of the invention, the nicotine container and/or the additive container comprises a pH-controlling agent, such as a buffering agent, being a non-salt pH-controlling agent, such as a non-salt buffering agent.

According to an advantageous embodiment of the invention, said pH-controlling agent comprises a Lewis acid and/or a Lewis base.

It may be advantageous according to embodiments of the invention to use pH-controlling agents that are soluble in organic carriers and/or excipients.

According to an advantageous embodiment of the invention, said Lewis base is selected from the group consisting of amines, ammonia and alcohols.

According to an advantageous embodiment of the invention, the container arrangement comprises a nicotine container and an additive container.

The nicotine container comprises nicotine and the additive container comprises additive.

According to an advantageous embodiment of the invention, the first atomizer produces aerosols on basis of nicotine-solution received from the nicotine container and where the second atomizer produces aerosols on basis of additive solution received from the additive container.

One advantage of the above embodiment may be that the two different kind of aerosols may be produced, one kind being nicotine-containing aerosols and the other kind being additive-containing aerosols, and that the two kind of aerosols may be directed to the lungs and the oral cavity, respectively, by means of different mass median aerodynamic diameters (MMAD) of the two kinds of aerosols.

According to an advantageous embodiment of the invention, the nicotine solution and/or the additive solution comprise one or more pharmaceutically acceptable excipients or carriers.

In certain embodiments, it may be preferred to use excipients or carriers, which, when atomized are visible to the human eye, whereby they imitate the appearance of smoke from conventional cigarettes.

An example of such a carrier aiding in creating a visible aerosol may be propylene glycol.

According to an advantageous embodiment of the invention, the pharmaceutically acceptable excipients or carriers are chosen from the group consisting of water; terpenes, such as menthol; alcohols, such as ethanol, propylene glycol, polyethylene glycol, such as PEG 400, glycerol and other similar alcohols; dimethylformamide; dimethylacetamide; wax; supercritical carbon dioxide; dry ice; and mixtures or combinations thereof.

According to an advantageous embodiment of the invention, the pharmaceutically acceptable excipients or carriers comprise propylene glycol.

According to an advantageous embodiment of the invention, the pharmaceutically acceptable excipients or carriers comprise PEG 400.

According to an advantageous embodiment of the invention, the pharmaceutically acceptable excipients or carriers comprise glycerol.

One advantage of the above embodiment may be that an effective delivery of said nicotine and said additive while said aerosols may be provided to appear smoke-like. Thereby, the user of the electronic nicotine delivery system may perceive the usage of the electronic nicotine delivery system to resemble conventional smoking, which may be a significant advantage for a user trying to stop smoking.

According to the invention, said nicotine container comprises nicotine in an amount of 0.01-5% by weight of the nicotine solution, such as 0.1-5% by weight of the nicotine solution.

According to an advantageous embodiment of the invention, the solution in said nicotine container and/or said additive container comprises glycerol in an amount of 0-95% by weight, such as 0.01-95% by weight, such as 0.1-95% by weight..

According to an advantageous embodiment of the invention, the solution in said nicotine container and/or said additive container comprises propylene glycol in an amount of 0-95% by weight, such as 0.01-95% by weight, such as 0.1-95% by weight.

According to an advantageous embodiment of the invention, the solution in said nicotine container and/or said additive container comprises 0.1-20% by weight of water, such as 0.1-15% by weight of water, such as 0-10% by weight of water, or such as 5-15% by weight of water.

According to an advantageous embodiment of the invention, the solution in said additive container comprises 0.01 - 10% by weight of flavoring, such as 0.01 - 5% by weight of flavoring, 0.01 - 0.5% by weight of flavoring.

According to an advantageous embodiment of the invention, the additive comprises one or more flavorings.

Typically, it may be desired that the user experiences one or more flavoring sensations when using the electronic nicotine delivery system. This may e.g. be done to mask the taste of nicotine. The flavorings may be designed to imitate a smoking experience of a conventional cigarette, or may be based on other flavorings, or may combine the two.

According to an advantageous embodiment of the invention, the one or more flavorings comprise almond, almond amaretto, apple, Bavarian cream, black cherry, black sesame seed, blueberry, brown sugar, bubblegum, butterscotch, cappuccino, caramel, caramel cappuccino, cheesecake (graham crust), cinnamon redhots, cotton candy, circus cotton candy, clove, coconut, coffee, clear coffee, double chocolate, energy cow, graham cracker, grape juice, green apple, Hawaiian punch, honey, Jamaican rum, Kentucky bourbon, kiwi, koolada, lemon, lemon lime, tobacco, maple syrup, maraschino cherry, marshmallow, menthol, milk chocolate, mocha, Mountain Dew, peanut butter, pecan, peppermint, raspberry, banana, ripe banana, root beer, RY 4, spearmint, strawberry, sweet cream, sweet tarts, sweetener, toasted almond, tobacco, tobacco blend, vanilla bean ice cream, vanilla cupcake, vanilla swirl, vanillin, waffle, Belgian waffle, watermelon, whipped cream, white chocolate, wintergreen, amaretto, banana cream, black walnut, blackberry, butter, butter rum, cherry, chocolate hazelnut, cinnamon roll, cola, creme de menthe, eggnog, English toffee, guava, lemonade, licorice, maple, mint chocolate chip, orange cream, peach, pina colada, pineapple, plum, pomegranate, pralines and cream, red licorice, salt water taffy, strawberry banana, strawberry kiwi, tropical punch, tutti frutti, vanilla, or any combination thereof.

A flavoring can be used to pair nicotine administration with certain gustatory and/or olfactory sensations. Subsequent administration of agent (e.g. nicotine) doses can be reduced while retaining the flavoring to help the user reduce their agent (e.g. nicotine) dependency.

According to an advantageous embodiment of the invention, the electronic nicotine delivery system is handheld.

The pH value of saliva in the oral cavity is throughout the application referred to the below measuring procedure.

Ten representative users of the nicotine delivery system in question are delivering saliva to the test. Puff duration is chosen to be 3 seconds. The puff velocity is given as 20 ml/seconds given a puff volume of 60 ml.

The pH in the saliva is measured by collecting 1 ml of saliva from the users in individual vials after 10 puffs and the pH is measured with a calibrated pH meter within two minutes from collecting the saliva. The ten puffs are performed by the individual users within 5 minutes.

Saliva must not be swallowed at any time but shall be collected in plastic vials.

The average pH value obtained from these measurements is taken as the representative pH value for the given nicotine delivery system.

### Figures

The invention will be described in the following with reference to the figures in which:
Figure 1 illustrates an electronic nicotine delivery system according to an embodiment,
figure 2A illustrates a part of an electronic nicotine delivery system according to an embodiment,
figure 2B illustrates a part of an electronic nicotine delivery system according to an embodiment,
figure 3 illustrates an electronic nicotine delivery system according to an embodiment, and
figure 4 illustrates an electronic nicotine delivery system according to an embodiment.

### Detailed description

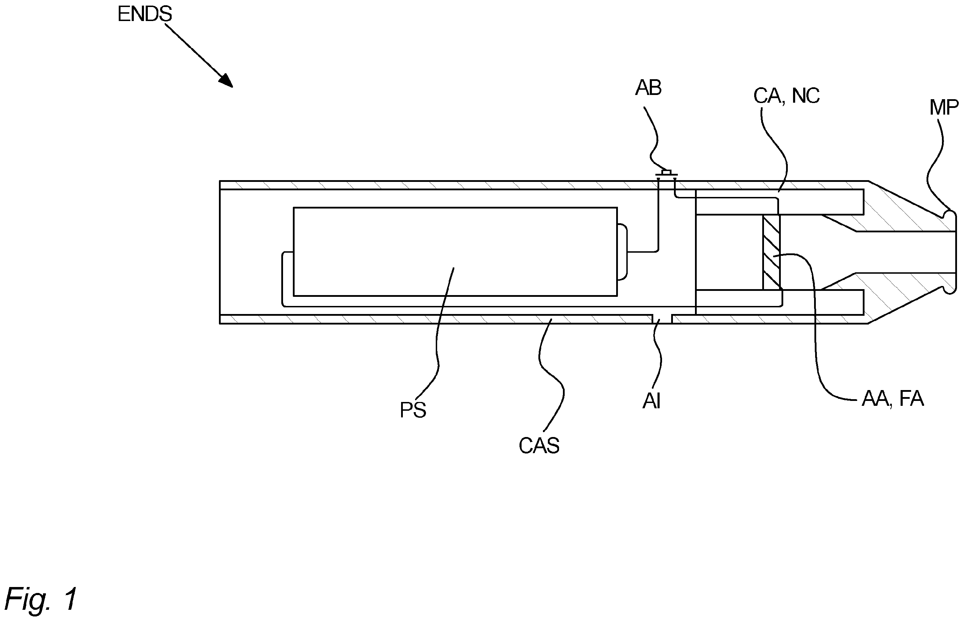

Referring to figure 1, an electronic nicotine delivery system ENDS is illustrated according to an embodiment of the invention. The electronic nicotine delivery system ENDS comprises a casing CAS for covering the individual parts of the electronic nicotine delivery system ENDS.

The casing CAS may be a single part, or may be assembled from two or more parts.

The electronic nicotine delivery system ENDS furthermore comprises a container arrangement CA and an atomizer arrangement AA.

In the present embodiments, the container arrangement CA comprises a nicotine container NC.

In some embodiments, the container arrangement CA may comprise further containers, such as an additive container AC.

The atomizer arrangement AA comprises a first atomizer FA. In some embodiments, the atomizer arrangement AA may comprise a second atomizer SA, and, optionally, even further atomizers.

The electronic nicotine delivery system ENDS furthermore comprises a mouth piece MP. The mouth piece MP is adapted for allowing a user of the electronic nicotine delivery system ENDS to apply a mouth vacuum to the electronic nicotine delivery system ENDS via suction at the mouth piece MP, i.e. when the user takes a drag or puff from the electronic nicotine delivery system ENDS similar to that from a conventional cigarette.

The casing CAS may preferably comprise one or more air inlets AI for supplying air to the atomizers FA, SA. The one or more air inlets AI may be positioned between the power source PS and the atomizer arrangement AA, or at other positions.

The atomizer FA may preferably be positioned in an inner air passage IAP. The inner air passage IAP may preferably provide fluid communication from said one or more air inlets AI to said mouth piece MP through the inside of said electronic nicotine delivery system ENDS.

The mouth piece MP comprises an opening into the inner part of the electronic nicotine delivery system ENDS, that opening being in fluid communication via the inside of said electronic nicotine delivery system ENDS to the air inlet AI, and, optionally, additional air inlets AAI (not shown) through said inner air passage IAP.

The nicotine container NC are positioned inside the casing CAS.

The nicotine container NC is connected to the first atomizer FA. Thereby, the content of the nicotine container NC is each allowed to move to the first atomizer FA to which it is connected.

Inside the casing CAS, a power source PS, such as a battery, is arranged. The power source PS is electrically connected to the first atomizer FA so as to power the first atomizer FA when it is activated. In this embodiment the first atomizer FA is shown comprising a transport element TE being a wick in fluid communication with the nicotine container NC and a heating element HE being a coil for heating and atomizing, when the first atomizer FA is activated. In alternative embodiments the first atomizer FA may comprise additional and/or alternative elements.

In embodiments where the electronic nicotine delivery system ENDS comprises a further container, such as an additive container AC, the additive container AC may preferably be connected to a separate atomizer, e.g. a second atomizer SA. However, in some embodiments, the nicotine container NC and further containers may be connected to the same atomizer FA.

The heating element HE may in other embodiments be other than a coil.

The transport element TE may in other embodiments be other than a wick.

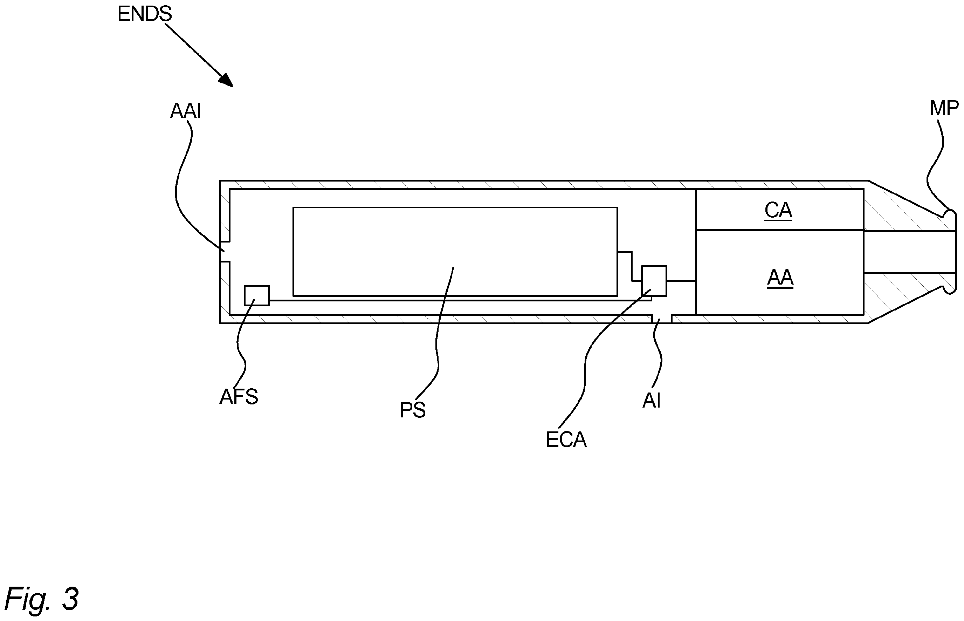

In this embodiments the electronic nicotine delivery system ENDS comprises an activator button AB for activating the atomizer FA. However, in alternative embodiments, the electronic nicotine delivery system ENDS may comprise other arrangements for activating the atomizer. For example, the electronic nicotine delivery system ENDS may comprise an air flow sensor AFS for detecting when a user applied a mouth generated vacuum to the mouth piece MP. This is illustrated on figure 3.

Returning to figure 1, the mouth piece MP may in some embodiments be detachable from the rest of the electronic nicotine delivery system ENDS, e.g. by means of threaded connections.

The nicotine container NC and/or further container(s), if any, may in some embodiments be removable and replaceable, preferably as a single cartridge, e.g. by removing the mouth piece MP and sliding the containers out by that end.

In some embodiments the one or more atomizers FA, SA is connected to the one or more containers NC, AC and thereby removed together with the containers NC, AC, e.g. as a single cartridge. However, in other embodiments, the containers NC, AC may be removed without the atomizers FA, SA, e.g. as a single cartridge.

In the following, electronic nicotine delivery systems ENDS according to various embodiments of the invention are illustrated. The electronic nicotine delivery systems ENDS of the following embodiments may comprise one or more elements similar to the elements described above. The electronic nicotine delivery systems ENDS of the following embodiments may comprise one or more elements additional or alternative to the elements described above.

Electrical connections are shown in the figures for illustrative purposes and may for practical purposes be arranged and positioned differently.

According to one embodiment of the invention, the nicotine container NC comprises a buffering agent for adjusting the pH-value. When the atomizer FA is activated, a part of the content of the nicotine container NC is atomized and the resulting aerosols are inhaled by the user of the electronic nicotine delivery system ENDS. By including a buffering agent in the nicotine container, along with nicotine, the part of the nicotine being absorbed in the oral cavity would be buffered by the accompanying buffering agent, whereby the degree of uptake of nicotine from the oral cavity may be increased.

In some other embodiments, the electronic nicotine delivery system ENDS may comprise one or more further containers, such as an additive container AC. In such embodiments, the nicotine container NC and/or the one or more further containers, such as the additive container AC, may comprise buffering agent for increasing the nicotine uptake through the oral mucosa.

Furthermore, in many embodiments of the invention, the electronic nicotine delivery system comprises an electrical control arrangement ECA. The electronic control arrangement ECA may comprise several co-operating different units, it may be comprised in one housing or it may even be integrated into other units, e.g. the power supply. The electronic control arrangement ECA is electrically connected to the atomizers and the activation arrangement, such as an activation button and/or an air flow sensor.

The electronic control arrangement ECA is arranged to controls the effective dose delivered by the atomizer on the basis of an automatic regulation of the electrical power supplied to the atomizer AT by the power supply PS and/or the activation time.

Furthermore, the electronic control arrangement ECA may in some embodiments with more than one atomizer be adapted to control the activation of the atomizers in a synchronized manner. In some embodiments, electronic control arrangement ECA may impose a delay of a predetermined period of time between the activation of the atomizers.

Furthermore, the electronic control arrangement ECA may in some embodiments control the dose supplied to the atomizer.

Furthermore, the electronic control arrangement ECA may in some embodiments may control the aerosol particle size of the aerosols produced by the atomizer.

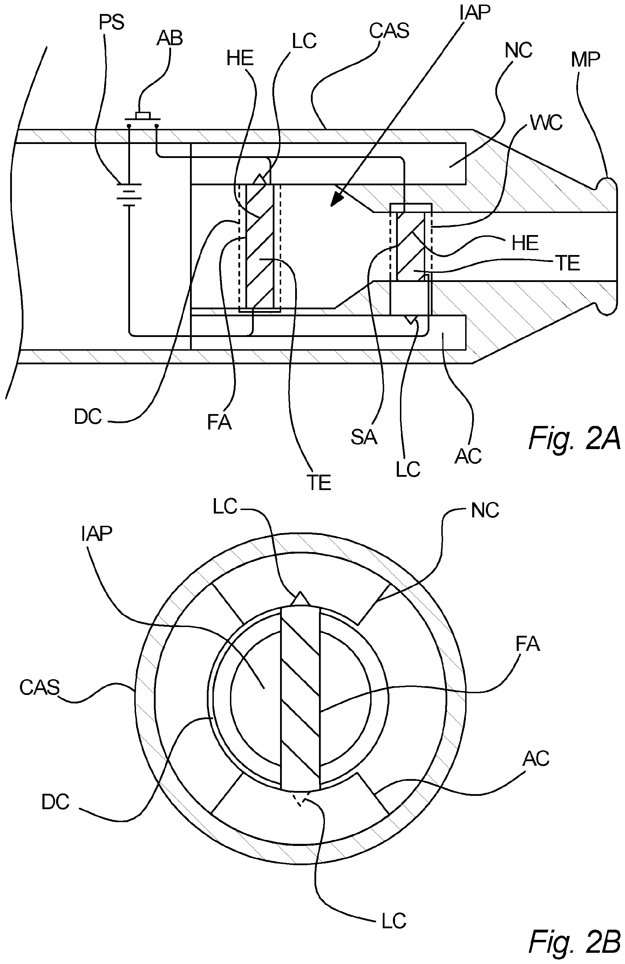

Referring to figure 2A and 2B, a part of an electronic nicotine delivery system ENDS is illustrated according to an embodiment of the invention. Figure 2A illustrates a partially cross-sectional side view, whereas figure 2B illustrates a cross-sectional end view, as seen from the left towards the right on figure 2A.

The electronic nicotine delivery system ENDS of the present embodiment is an alternative to the embodiment of figure 1.

The electronic nicotine delivery system ENDS comprises a nicotine container NC as well as an additive container AC:
The electronic nicotine delivery system ENDS furthermore comprises a first atomizer FA and a second atomizer SA.

Preferably, as illustrated on figure 2, the first atomizer FA is connected to the nicotine container NC so as to produce nicotine-containing aerosols based on the content of the nicotine container NC.

Preferably, as illustrated on figure 2, the second atomizer SA is connected to the additive container NC so as to produce additive-containing aerosols based on the content of the additive container AC.

The nicotine container NC and/or the additive container AC may comprise the pH-controlling agent.

According to a preferred embodiment, the additive container AC comprises a pH-controlling agent for adjusting the pH-value on the oral cavity, whereby the nicotine uptake through the oral mucosa is increased.

The first and second atomizers FA, SA are longitudinally displaced inside the inner air passage IAP such that the diameter of the inner air passage IAP is different at each atomizer FA, SA. Thereby, since the total flow rate is constant over the inner air passage IAP, the flow velocity at the first atomizer FA is lower that the flow velocity at the second atomizer SA, due to the cross-sectional flow area being smaller at the second atomizer SA compared to at the first atomizer FA. Different air flow velocity at the respective atomizers FA, SA may, together with other result-effective parameters, determine the aerosol particle size of the aerosols from the respective atomizer FA, SA.

The first and second atomizers FA, SA are in this embodiment illustrated having a transport element TE being a wick and a heating element HE being a coil arranged around a part of the wick. When the coil is heated, it provides resistive heating by means of a power source PS. In alternative embodiments the atomizers FA, SA may comprise additional and/or alternative elements. In some alternative embodiments, the heating element HE may be e.g. a plate or a tube, heated e.g. by resistive heating. In some alternative embodiments, the transport element TE may comprise e.g. a tube, such as a capillary tube, and/or may comprise a pump, such as an electronic pump.

In some alternative embodiments, the atomizers may not comprise heating elements HE.

In some alternative embodiments, only one atomizer comprise a heating element HE. Preferable, the only one atomizer is the first atomizer FA producing nicotine-containing aerosols.

Moreover figure 2B illustrates that nicotine container NC and the additive container AC each are positioned about the inner air passage IAP in which the atomizers FA, SA are positioned.

Preferably, as illustrated, the wick of the first atomizer FA is in fluid communication with the nicotine container NC. Preferably, both ends of each wick are in fluid communication with their respective containers. This may be facilitated by the first atomizer FA comprising a distribution conduit DC providing fluid communication from the end of the wick disposed near the nicotine container NC to the opposite end. The second atomizer may be constructed in a similar way.

Each atomizer FA, SA may comprise a distribution conduit DC for transporting the content of the respective container NC, AC to the end of the wick facing away from the respective container NC, AC. Thereby, a more uniform wetting, or distribution of the container content over the length of the wick may be obtained. Also, a faster transport of container content to and throughout the wick after one activation of the respective atomizer FA, SA to the next activation may be obtained, i.e. a faster reload after the user activates one or both atomizers FA, SA.

The electronic nicotine delivery system ENDS may furthermore comprise a liquid coupling LC for coupling liquid from the nicotine container NC or the additive container AC to the first and second atomizers FA, SA, respectively. The liquid coupling may in some embodiments be arranged to pierce a part of the relevant container NC, AC to provide access and liquid communication from the inside of the respective container NC, AC to the outside of that container NC, AC.

In figures 2A and 2B the wicks of the first and second atomizers FA, SA are shown as substantially parallel, which is why the second atomizer SA is hidden behind the first atomizer FA in figure 2B. However, in other embodiments, the two atomizers FA, SA may be oriented with an angle relative to each other, when seen from the end as in figure 2B, e.g. 90° (degrees).

Now, referring to figure 3 an electronic nicotine delivery system ENDS is illustrated according to a further embodiment of the invention. The electronic nicotine delivery system ENDS comprises a casing CAS with a mouth piece MP, a power source PS, such as a battery, an air flow sensor AFS, an electronic control arrangement ECA, an atomizer arrangement AA, and a container arrangement CA. The casing CAS comprises an air inlet AI and an additional air inlet. Each air inlet AI, AAI is in fluid communication mouth piece MP through the inside of the electronic nicotine delivery system ENDS so as to provide air when a user applies a reduced pressure to the mouth piece MP.

The atomizer arrangement AA may be arranged according to any of the embodiments described in relation to figure 1 or 2. Specifically, the atomizer arrangement AA may in some embodiments, only a first atomizer FA, whereas the atomizer arrangement AA in other embodiments may comprise both a first and a second atomizer FA, SA.

The container arrangement CA may be arranged according to any of the embodiments described in relation to figure 1 or 2. Specifically, the container arrangement may in some embodiments comprise only a nicotine container NC, whereas the container arrangement CA may in other embodiments comprise both a nicotine container NC and an additive container AC.

In some embodiments the air inlet AI is the primary air inlet, providing e.g. at least 70% of the air, such as at least 80%, such as at least 90%, such as at least 95%.

The air flow sensor AFS may be positioned near the additional air inlet AAI so as to detect air flow through the additional air inlet AAI, which is indicative of a user applying a reduced pressure to the mouth piece MP. Alternatively, the air flow sensor AFS may be positioned air inlet AI, whereby the additional air inlet AAI in some cases may be disposed of.

When the air flow sensor AFS detects air flow, it sends a signal to the electronic control arrangement ECA which activates the atomizer arrangement AA, e.g. by activating the power to the atomizer arrangement AA.

Thereby, when the user applies a reduced pressure to the mouth piece MP, the atomizer arrangement AA may be automatically activated.

In some embodiments, the electronic nicotine delivery system ENDS may further to the air flow sensor AFS comprise an activator button (not shown). In such cases, the activator button AB may be used to determine the dose delivered from the nicotine container NC and/or the additive container AC, e.g. determined from the temporal length of the button activation. Alternatively, the strength of the reduced pressure applied to the mouth piece MP and detected by the air flow sensor AFS may determine the dose delivered from the nicotine and/or the additive container.

In a further embodiment, only one atomizer FA, SA is activated automatically by means of the air flow sensor AFS, whereas the other atomizer FA, SA must be activated by the activator button AB. Preferably, it may be the first atomizer FA connected to the nicotine container NC that must be activated via the activator button AB.

The present embodiment may be employed on connection with container and atomizer designs illustrated on figures 1-2.

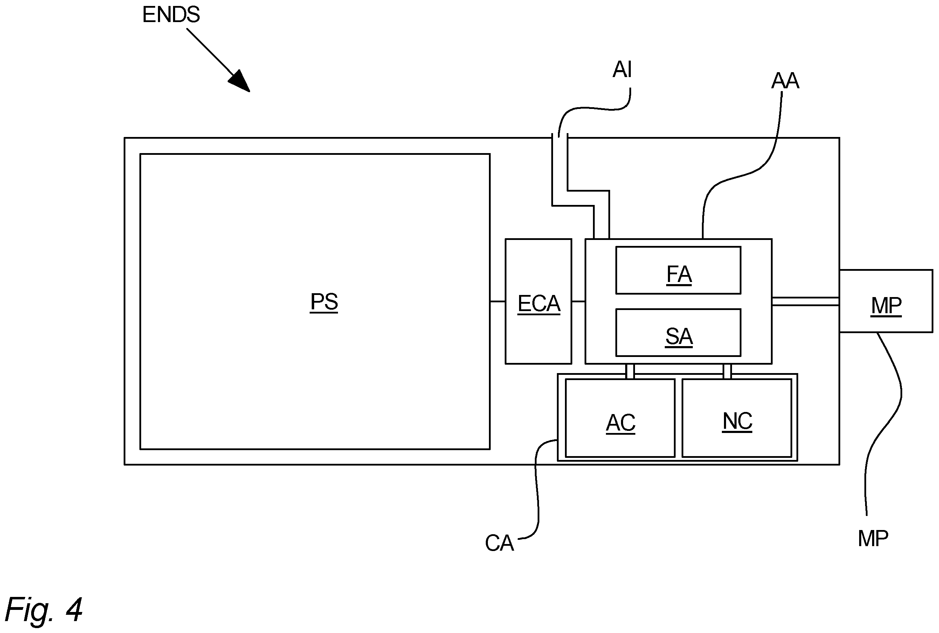

Now referring to figure 4, an electronic nicotine delivery system ENDS according to a further embodiment of the invention is illustrated.

The electronic nicotine delivery system ENDS comprises a power supply PS, such as a battery. Typically, the power supply PS may take up a substantial part of the electronic nicotine delivery system ENDS.

The electronic nicotine delivery system ENDS furthermore comprises an atomizer arrangement AA.

The atomizer arrangement AA may preferably, as illustrated, comprise a first atomizer FA and a second atomizer SA. Alternatively, the atomizer arrangement AA may comprise only a first atomizer FA, similar to the embodiments illustrated in relation to figure 1.

The atomizers FA, SA are electrically connected to the power supply PS. The atomizers FA, SA may be constructed similar to the aforementioned embodiments illustrated on figures 1-3. The first second atomizer FA may preferably be constructed in substantially the same way as the second atomizer SA.

The electronic nicotine delivery system ENDS furthermore comprises a container arrangement CA.

The container arrangement CA may preferably, as illustrated, comprise a nicotine container NC and an additive container AC. Alternatively, the container arrangement CA comprises only a nicotine container NC, similar to the embodiment illustrated in relation to figure 1.

The electronic nicotine delivery system ENDS furthermore comprises a mouth piece MP for a user to apply an orally generated reduced pressure to and for the user to received aerosolized content of the nicotine container NC and/or the additive container AC. The mouth piece MP is in fluid communication with the atomizers FA, SA inside said electronic nicotine delivery system ENDS for facilitating transport of aerosols from the atomizers FA, SA.

The electronic nicotine delivery system ENDS may furthermore comprise one or more air inlets AI. The air inlet AI is in fluid communication with the atomizer arrangement AA inside the electronic nicotine delivery system ENDS, thereby facilitating transport of air from the air inlet AI to the atomizer arrangement AA.

The electronic nicotine delivery system ENDS may furthermore comprise an electronic control arrangement ECA.

The electronic control arrangement may preferably be powered by the power supply PS.

The electronic control arrangement ECA may control the activation of the atomizers FA, SA based on inputs from a user of the electronic nicotine delivery system ENDS. Such user inputs may comprise a signal from an activation button (not shown) activated by the user and/or detection of user application of orally generated reduced pressure to the mouth piece MP, e.g. by means of an air flow sensor AFS (not shown).

The electronic control arrangement ECA may activate the first and second atomizer FA, SA simultaneously, or delay the activation of the first or second atomizer FA, SA relative to the other atomizer FA, SA with a predetermined period of time.

The electronic control arrangement ECA may activate the first and second atomizer FA, SA for approximately the same period of time, or extend the activation of the first or second atomizer FA, SA if needed.

### List of figure references

ENDS. Electronic nicotine delivery system
MP. Mouth piece
AA. Atomizer arrangement
PS. Power supply
NC. Nicotine container
AC. Additive container
FA. First atomizer
SA. Second atomizer
TE. Transport element
HE. Heating element
CAS. Casing
AB. Activator button
AI. Air inlet
AAI. Additional air inlet
AFS. Air flow sensor
ECA. Electronic control arrangement
DC. Distribution conduit
IAP. Inner air passage
LC. Liquid coupling
CA. Container arrangement

## Claims

1. Electronic nicotine delivery system (ENDS) comprising a mouth piece (MP), an atomizer arrangement (AA), a power supply (PS), and a container arrangement (CA) comprising a nicotine container containing a nicotine solution,
the atomizer arrangement (AA) comprising an inlet from the container arrangement (CA),
the atomizer arrangement (AA) comprising at least one atomizer,
the atomizer being electrically connected to the power supply (PS),
the container arrangement (CA) further containing pH-controlling agent in solution,
wherein the nicotine container comprises nicotine in an amount of 0.01-5% by weight of the nicotine solution,
and the atomizer arrangement producing aerosols comprising pH-controlling agent and nicotine,
wherein the pH-controlling agent comprises an acidic buffering agent, **characterized in that** the container arrangement comprises buffering agent in the amount of ½ to 5% by weight of the total content of said container arrangement.

2. Electronic nicotine delivery system (ENDS) according to claim 1, wherein the pH-controlling agent is selected to optimize the nicotine uptake via the oral mucosa of a user when the pH-controlling agent comprised in an aerosol produced by the atomizer arrangement is delivered to the oral cavity of a user.

3. Electronic nicotine delivery system (ENDS) according to claim 1 or 2, wherein the container arrangement (CA) comprises an additive container (AC) comprising pH-controlling agent.

4. Electronic nicotine delivery system (ENDS) according to any of the preceding claims, wherein the additive container (AC) comprises flavoring.

5. Electronic nicotine delivery system (ENDS) according to any of the preceding claims, wherein the atomizer comprises a heating element (HE).

6. Electronic nicotine delivery system (ENDS) according to any of the preceding claims, wherein the nicotine container and/or the additive container comprises a pH-controlling agent, such as a buffering agent, being a non-salt pH-controlling agent, such as a non-salt buffering agent.

7. Electronic nicotine delivery system (ENDS) according to any of the preceding claims, wherein said pH-controlling agent comprises a Lewis acid and/or a Lewis base.

8. Electronic nicotine delivery system (ENDS) according to any of the preceding claims, wherein the container arrangement (CA) comprises a nicotine container (NC) and an additive container (AC).

9. Electronic nicotine delivery system (ENDS) according to claim 8, wherein the first atomizer (FA) produces aerosols on basis of nicotine-solution received from the nicotine container (NC) and where the second atomizer (FA) produces aerosols on basis of additive solution received from the additive container (NC).

10. Electronic nicotine delivery system (ENDS) according to any of the preceding claims, wherein the nicotine solution and/or the additive solution comprises one or more pharmaceutically acceptable excipients or carriers such as pharmaceutically acceptable excipients or carriers chosen from the group consisting of water; terpenes, such as menthol; alcohols, such as ethanol, propylene glycol, polyethylene glycol, such as PEG 400, glycerol and other similar alcohols; dimethylformamide; dimethylacetamide; wax; supercritical carbon dioxide; dry ice; and mixtures or combinations thereof.

11. Electronic nicotine delivery system (ENDS) according to any of the preceding claims, wherein said nicotine container (NC) comprises nicotine in an amount of 0.1-5% by weight of the nicotine solution.

12. Electronic nicotine delivery system (ENDS) according to any of the preceding claims, wherein the solution in said nicotine container (NC) and/or said additive container (AC) comprises glycerol in an amount of 0-95% by weight, such as 0.01-95% by weight, such as 0.1-95% by weight.

13. Electronic nicotine delivery system (ENDS) according to any of the preceding claims, wherein the solution in said additive container (AC) comprises 0.01 - 10% by weight of flavoring, such as 0.01 - 5% by weight of flavoring, 0.01 - 0.5% by weight of flavoring.

14. Electronic nicotine delivery system (ENDS) according to any of the preceding claims, wherein the additive comprises one or more flavorings.

15. Electronic nicotine delivery system (ENDS) according to any of the preceding claims, wherein the electronic nicotine delivery system (ENDS) is handheld.

## Patentansprüche

1. Elektronisches Nikotinabgabesystem (ENDS), umfassend ein Mundstück (MP), eine Zerstäuberanordnung (AA), eine Stromversorgung (PS) und eine Behälteranordnung (CA), umfassend einen Nikotinbehälter, der eine Nikotinlösung enthält,
die Zerstäuberanordnung (AA) umfassend einen Einlass von der Behälteranordnung (CA),
die Zerstäuberanordnung (AA) umfassend mindestens einen Zerstäuber,
wobei der Zerstäuber mit der Stromversorgung (PS) elektrisch verbunden ist,
wobei die Behälteranordnung (CA) ferner ein pH-Regulierungsmittel in Lösung enthält, wobei der Nikotinbehälter Nikotin in einer Menge von 0,01 - 5 Gew.-% der Nikotinlösung umfasst,
und die Aerosole erzeugende Zerstäuberanordnung umfassend das pH-Regulierungsmittel und Nikotin,
wobei das pH-Regulierungsmittel eine saure Puffersubstanz umfasst, **dadurch gekennzeichnet, dass** die Behälteranordnung die Puffersubstanz in der Menge von ½ bis 5 Gew.-% des Gesamtgehalts der Behälteranordnung umfasst.

2. Elektronisches Nikotinabgabesystem (ENDS) nach Anspruch 1, wobei das pH-Regulierungsmittel ausgewählt ist, um die Nikotinaufnahme über die Mundschleimhaut eines Benutzers zu optimieren, wenn das pH-Regulierungsmittel, das in einem Aerosol beinhaltet ist, das durch die Zerstäuberanordnung erzeugt wird, an die Mundhöhle eines Benutzers abgegeben wird.

3. Elektronisches Nikotinabgabesystem (ENDS) nach Anspruch 1 oder 2, wobei die Behälteranordnung (CA) einen Additivbehälter (AC) umfasst, umfassend das pH-Regulierungsmittel.

4. Elektronisches Nikotinabgabesystem (ENDS) nach einem der vorstehenden Ansprüche, wobei der Additivbehälter (AC) einen Aromastoff umfasst.

5. Elektronisches Nikotinabgabesystem (ENDS) nach einem der vorstehenden Ansprüche, wobei der Zerstäuber ein Heizelement (HE) umfasst.

6. Elektronisches Nikotinabgabesystem (ENDS) nach einem der vorstehenden Ansprüche, wobei der Nikotinbehälter und/oder der Additivbehälter ein pH-Regulierungsmittel, wie eine Puffersubstanz, umfasst, die ein Nicht-SalzpH-Regulierungsmittel ist, wie eine Nicht-Salzpuffersubstanz.

7. Elektronisches Nikotinabgabesystem (ENDS) nach einem der vorstehenden Ansprüche, wobei das pH-Regulierungsmittel eine Lewis-Säure und/oder eine Lewis-Base umfasst.

8. Elektronisches Nikotinabgabesystem (ENDS) nach einem der vorstehenden Ansprüche, wobei die Behälteranordnung (CA) einen Nikotinbehälter (NC) und einen Additivbehälter (AC) umfasst.

9. Elektronisches Nikotinabgabesystem (ENDS) nach Anspruch 8, wobei der erste Zerstäuber (FA) auf Basis der von dem Nikotinbehälter (NC) empfangenen Nikotinlösung Aerosole erzeugt und wobei der zweite Zerstäuber (FA) auf Basis der von dem Additivbehälter (NC) empfangenen Additivlösung Aerosole erzeugt.

10. Elektronisches Nikotinabgabesystem (ENDS) nach einem der vorstehenden Ansprüche, wobei die Nikotinlösung und/oder die Additivlösung einen oder mehrere pharmazeutisch verträgliche Hilfsstoffe oder Träger, wie pharmazeutisch verträgliche Hilfsstoffe oder Träger, umfasst, die aus der Gruppe ausgewählt sind, bestehend aus Wasser; Terpenen, wie Menthol; Alkoholen, wie Ethanol, Propylenglykol, Polyethylenglykol, wie PEG 400, Glyzerin und anderen ähnlichen Alkoholen; Dimethylformamid; Dimethylazetamid; Wachs; überkritischem Kohlendioxid; Trockeneis; und Mischungen oder Kombinationen davon.

11. Elektronisches Nikotinabgabesystem (ENDS) nach einem der vorstehenden Ansprüche, wobei der Nikotinbehälter (NC) Nikotin in einer Menge von 0,1-5 Gew.-% der Nikotinlösung umfasst.

12. Elektronisches Nikotinabgabesystem (ENDS) nach einem der vorstehenden Ansprüche, wobei die Lösung in dem Nikotinbehälter (NC) und/oder dem Additivbehälter (AC) Glyzerin in einer Menge von 0-95 Gew.-%, wie 0,01 - 95 Gew.-%, wie 0,1-95 Gew.-%, umfasst.

13. Elektronisches Nikotinabgabesystem (ENDS) nach einem der vorstehenden Ansprüche, wobei die Lösung in dem Additivbehälter (AC) 0,01 bis 10 Gew.-% Aromastoff, wie 0,01-5 Gew.-% Aromastoff, 0,01-0,5 Gew.-% Aromastoff, umfasst.

14. Elektronisches Nikotinabgabesystem (ENDS) nach einem der vorstehenden Ansprüche, wobei das Additiv einen oder mehrere Aromastoffe umfasst.

15. Elektronisches Nikotinabgabesystem (ENDS) nach einem der vorstehenden Ansprüche, wobei das elektronische Nikotinabgabesystem (ENDS) tragbar ist.

## Revendications

1. Système électronique de distribution de nicotine (ENDS) comprenant un bec (MP), un agencement d'atomiseur (AA), une alimentation électrique (PS) et un agencement de récipient (CA) comprenant un récipient de nicotine contenant une solution de nicotine,
l'agencement d'atomiseur (AA) comprenant une entrée provenant de l'agencement de récipient (CA),
l'agencement d'atomiseur (AA) comprenant au moins un atomiseur,
l'atomiseur étant connecté électriquement à l'alimentation électrique (PS),
l'agencement de récipient (CA) contenant en outre un agent de régulation de pH en solution, le récipient de nicotine comprenant de la nicotine en une quantité de 0,01 à 5 % en poids de la solution de nicotine,
et l'agencement d'atomiseur produisant des aérosols comprenant un agent de régulation de pH et de la nicotine,
l'agent de régulation de pH comprenant un agent tampon acide, **caractérisé en ce que** l'agencement de récipient comprend un agent tampon dans la quantité de 1/2 à 5 % en poids de la teneur totale dudit agencement de récipient.

2. Système électronique de distribution de nicotine (ENDS) selon la revendication 1, dans lequel l'agent de régulation de pH est sélectionné pour optimiser l'absorption de nicotine par l'intermédiaire de la muqueuse buccale d'un utilisateur lorsque l'agent de régulation de pH compris dans un aérosol produit par l'agencement d'atomiseur est délivré à la cavité buccale d'un utilisateur.

3. Système électronique de distribution de nicotine (ENDS) selon la revendication 1 ou 2, dans lequel l'agencement de récipient (CA) comprend un récipient d'additif (AC) comprenant un agent de régulation de pH.

4. Système électronique de distribution de nicotine (ENDS) selon l'une quelconque des revendications précédentes, dans lequel le récipient d'additif (AC) comprend un arôme.

5. Système électronique de distribution de nicotine (ENDS) selon l'une quelconque des revendications précédentes, dans lequel l'atomiseur comprend un élément chauffant (HE).

6. Système électronique de distribution de nicotine (ENDS) selon l'une quelconque des revendications précédentes, dans lequel le récipient de nicotine et/ou le récipient d'additif comprennent un agent de régulation de pH, tel qu'un agent tampon, étant un agent de régulation de pH non salin, tel qu'un agent tampon non salin.

7. Système électronique de distribution de nicotine (ENDS) selon l'une quelconque des revendications précédentes, dans lequel ledit agent de régulation de pH comprend un acide de Lewis et/ou une base de Lewis.

8. Système électronique de distribution de nicotine (ENDS) selon l'une quelconque des revendications précédentes, dans lequel l'agencement de récipient (CA) comprend un récipient de nicotine (NC) et un récipient d'additif (AC).

9. Système électronique de distribution de nicotine (ENDS) selon la revendication 8, dans lequel le premier atomiseur (FA) produit des aérosols sur la base de la solution de nicotine reçue du récipient de nicotine (NC) et où le second atomiseur (FA) produit des aérosols sur la base de la solution additive reçue du récipient d'additif (NC).

10. Système électronique de distribution de nicotine (ENDS) selon l'une quelconque des revendications précédentes, dans lequel la solution de nicotine et/ou la solution d'additif comprennent un ou plusieurs excipients ou véhicules pharmaceutiquement acceptables tels que des excipients ou véhicules pharmaceutiquement acceptables choisis dans le groupe constitué d'eau ; les terpènes, tels que le menthol ; les alcools, tels que l'éthanol, le propylène glycol, le polyéthylène glycol, tel que le PEG 400, le glycérol et d'autres alcools similaires ; du diméthylformamide ; du diméthylacétamide ; de la cire ; du dioxyde de carbone supercritique ; de la glace carbonique ; et des mélanges ou des combinaisons de ceux-ci.

11. Système électronique de distribution de nicotine (ENDS) selon l'une quelconque des revendications précédentes, dans lequel ledit récipient de nicotine (NC) comprend de la nicotine en une quantité de 0,1 à 5 % en poids de la solution de nicotine.

12. Système électronique de distribution de nicotine (ENDS) selon l'une quelconque des revendications précédentes, dans lequel la solution dans ledit récipient de nicotine (NC) et/ou ledit récipient d'additif (AC) comprend du glycérol en une quantité de 0 à 95 % en poids, telle que 0,01 à 95 % en poids, telle que 0,1 à 95 % en poids.

13. Système électronique de distribution de nicotine (ENDS) selon l'une quelconque des revendications précédentes, dans lequel la solution dans ledit récipient d'additif (AC) comprend de 0,01 à 10 % en poids d'arôme, telle que 0,01 à 5 % en poids d'arôme, 0,01 à 0,5 % en poids d'arôme.

14. Système électronique de distribution de nicotine (ENDS) selon l'une quelconque des revendications précédentes, dans lequel l'additif comprend un ou plusieurs arômes.

15. Système électronique de distribution de nicotine (ENDS) selon l'une quelconque des revendications précédentes, dans lequel le système électronique de distribution de nicotine (ENDS) est portatif.
